# EUROPEAN PATENT SPECIFICATION

(11) **EP 3 494 877 B1**
(45) Date of publication and mention of the grant of the patent: **07.02.2024**
(21) Application number: 17206438.8
(22) Date of filing: 11.12.2017
(51) Int. Cl.: C12M 3/06, A61B 5/24

(54) **DEVICE FOR THE EXAMINATION OF NEURONS**
VORRICHTUNG ZUR UNTERSUCHUNG VON NEURONEN
DISPOSITIF D'EXAMEN DES NEURONES

(43) Date of publication of application: 12.06.2019
(73) Proprietor: NMI NATURWISSENSCHAFTLICHES UND MEDIZINISCHES INSTITUT AN DER UNIVERSITÄT TÜBINGEN, 72770 Reutlingen (DE)
(72) Inventor: Cesare, Paolo, 72074 Tübingen (DE); Jones, Peter, 72076 Tübingen (DE); Martinez, Beatriz Molina, 72072 Tübingen (DE)
(74) Representative: Witte, Weller & Partner Patentanwälte mbB

(56) References cited:
- WO-A1-2015/010305
- WO-A1-2015/092141
- RU-C1- 2 637 391
- BRADLEY J. DWORAK ET AL: "Novel MEA platform with PDMS microtunnels enables the detection of action potential propagation from isolated axons in culture", LAB ON A CHIP, vol. 9, no. 3, 18 November 2009 (2009-11-18), pages 404-410, XP055478313, ISSN: 1473-0197, DOI: 10.1039/B806689B
- HILLARY R IRONS ET AL: "Three-dimensional neural constructs: a novel platform for neurophysiological investigation; Engineered 3D neural constructs", JOURNAL OF NEURAL ENGINEERING, INSTITUTE OF PHYSICS PUBLISHING, BRISTOL, GB, vol. 5, no. 3, 15 September 2008 (2008-09-15), pages 333-341, XP020147193, ISSN: 1741-2552, DOI: 10.1088/1741-2560/5/3/006
- LIANGBIN PAN ET AL: "Propagation of action potential activity in a predefined microtunnel neural network;Propagation of action potential activity in a predefined microtunnel neural network", JOURNAL OF NEURAL ENGINEERING, INSTITUTE OF PHYSICS PUBLISHING, BRISTOL, GB, vol. 8, no. 4, 13 July 2011 (2011-07-13), page 46031, XP020209276, ISSN: 1741-2552, DOI: 10.1088/1741-2560/8/4/046031

## Description

The present invention relates to a device for the examination of neurons and to a method for examining neurons, and more specifically for examining neurons growing in a three-dimensional network.

Dysfunctions of the central nervous system represent a major burden for society and healthcare worldwide, affecting hundreds million people every year, with many patients obtaining little or no relief from current treatments.

The identification of new drugs to safely and effectively target specific neuronal circuits represents one of the main challenges that the pharmaceutical industry needs to face for the development of new therapeutic strategies to treat human brain disorders, in particular neurodegenerative diseases. To avoid late, high attrition rates and making drug development more cost effective, potential neurotoxicity and seizure liability has to be recognized as early as possible during the discovery process.

Assessment of the potential impact of new compounds on the nervous system represents a major effort also for the agro-chemical and the consumer products industry, as these studies are required by regulatory authorities before new chemical entities can be introduced into the market.

Most of these studies rely on the use of *ex vivo* and *in vivo* animal models, which are not always predictive of adverse events in humans. They are costly and time consuming and not amenable to high throughput testing of chemicals and compounds. In addition, they are ethically questionable due to the high number of animals needed and the impact on their wellness during substance exposure.

According to a recent policy in the European Union (Regulation, Evaluation Authorisation and Restriction of Chemicals, REACH), it has been estimated that over the next twelve to fifteen years, approximately 30,000 chemicals may need to be tested for safety, and under current guidelines such testing would require the use of approximately 7.2 million laboratory animals. It has also been estimated that out of 5,000 to 10,000 new drug entities that a pharmaceutical company may start with, only one is finally approved by the Food and Drug Administration at a cost of over one billion dollars. A large portion of this cost is due to animal testing.

Therefore, EU and international policies increasingly call for the reduction or replacement of *in vivo* animal studies in the process of drug development and toxicity testing. Thus, alternative *in vitro* approaches to hazard identification are needed that have higher throughput capabilities and are more predictive of *in vivo* effects.

Due to their intrinsic capacity to non-invasively measure the extracellular potential of hundreds or thousands of cells in parallel, in the last few years microelectrode array (MEA) recordings have become increasingly common for a variety of applications focusing on the study of neuronal networks and, more in general, excitable cells such as neurons and cardiomyocytes. This applies to both basic research and industries looking for a high-throughput, high-content assay for preclinical discovery, safety pharmacology and toxicology.

Several international groups have demonstrated that MEA recordings are very sensitive in detecting electrophysiological changes in neuronal networks. Thus MEA recordings provide a robust assay for efficacy, seizure liability and neurotoxicity testing in a variety of *in vitro* models. A large international consortium (CiPA Initiative), composed by public and industrial stakeholders, is evaluating MEA technology also for its possible application in the field of cardiac safety pharmacology. There, MEA technology in combination with the use of human iPSC-derived cardiomyocytes would offer an alternative to expensive and time-consuming *in vivo* animal models currently required by regulatory authorities.

Cells grown adherent on two-dimensional (2D) substrates do not fully represent true *in vivo* cell environments and lack extracellular components, cell-cell and cell-matrix interactions necessary for differentiation, proliferation and cell-based functions. More recently, three-dimensional (3D) cell culture techniques have been explored as a new opportunity in the biomedical research community. 3D cell culture techniques have demonstrated the potential to develop complex tissue structures *in vitro.* There is increasing evidence that 3D culture systems can capture important components of the complex physiology of a tissue or an organ better than classical monolayer approaches.

This brings the hope of lowering lead molecule attrition rates while enhancing overall predictability of *in vitro* assays. However, these models restrict huge data reproducibility, challenge assay readout systems and often require complex microfabrication processes. New developments in this direction, especially in throughput capabilities and disease modelling, have a great value for the identification and validation of novel targets involved in neuronal functions and dysfunctions.

In an effort to accelerate and increase the success rate of the drug-discovery process, the potential of combining and interfacing microtechnologies with brain cell populations, for example derived from human induced pluripotent stem cells (iPSCs), has recently become the focus of many pre-clinical research programs worldwide, for example at NIH (National Institutes of Health) and DARPA (Defense Advanced Research Projects Agency) in the U.S. By taking advantage of the human origin of iPSCs such new methods aim to improve predictability of a range of physiological, pharmacological and toxicological assays. To do so, several labs are attempting to create organ- and body-on-chip, looking at the possibility to generate 3D structures which, by mimicking the multicellular architecture of living organs, may better recapitulate human health and diseases.

However, as yet, there is no integrated, human relevant, *in vitro* 3D system or technology for assessing efficacy, neurotoxicity or seizure liability of new compounds based on the inherent physiological characteristics of the brain (i.e. electrical activity, tissue architecture, synaptic structures) that is fit for purpose for use in basic or applied research.

Brewer et al. (2013), Toward a self-wired active reconstruction of the hippocampal trisynaptic loop: DG-CA3, Front. Neural. Circuits., Vol. 7, Art. 165, p. 1-8, and Gladkov et al. (2017), Design of Cultured Neuron Networks in vitro with Predefined Connectivity Using Asymmetric Microfluidic Channels". Sci Rep. 7(1): 15625, disclose two-compartment neuron cultivation system, where both compartments are connected via microtunnels of different designs allowing the passing-through of adherent neurites. Such system is arranged on a multi-electrode array (MEA) comprising recording electrodes. Electrical activity of neurites was recorded by microelectrodes located within microtunnels, as is expected for cultured neurons adherent on MEA surfaces.

Tsantoulas et al. (2013), Probing functional properties of nociceptive axons using a microfluidic culture system, PLoS One, Vol. 8, Issue 11, p. 1-17, disclose a two-compartment neuron cultivation system, where both compartments are connected via microgrooves allowing the passing-through of neurites. Again, neurons were cultured adherent on the device surface. In the system basically a chemical stimulation is realized. In one variant an electrical stimulation occurs. A so-called field potential stimulation takes place via macroscopic "electrodes" in the form of simple wires placed into the cultivation compartment. Activity of neurons is mainly read out by calcium imaging, which only indirectly estimates electrical activity and does not match the temporal resolution of electrical recording. In addition, activity of neurons is recorded via the patch-clamp method, which is invasive and has negative effects on the architecture and integrity of the neurons.

The WO 2004/034016 discloses a two-compartment microfluidic device where the two compartments are coupled by a barrier comprising micron sized grooves. It is mentioned that positive or negative stimuli may be selectively applied to distal portions of the neurites, growing adherently to one of the surfaces of the device, at the point of the barrier with the grooves or channels, respectively.

The US 2004/230270 discloses an interface for selectively making electrical contact to a plurality of neural cells in a biological neural network, wherein said biological network comprises a brain cortex or retinal neural network. It is mentioned that spatially resolved electrical contact to neural cells can be made by allowing migration of cell soma into microchannels.

Further devices for the examination of neurons are known from Navarro et al. (2005), A critical review of interfaces with the peripheral nervous system for the control of neuroprostheses and hybrid bionic systems. J. Peripher. Nerv. Syst. 10(3):229-58; Zhuang et al. (2017), 3D neural tissue models: From spheroids to bioprinting. Biomaterials 154:113-133; Wang et al. (2012), Biophysics of microchannel-enabled neuron-electrode interfaces. J. Neural. Eng. 9(2):026010; Fitzgerald et al. (2008), Microchannels as axonal amplifiers. IEEE Trans. Biomed. Eng. 55(3):1136-46; US 3,955,560; EP 2 249 915; US 2017/311,827.

Further devices for the examination of neurons are also disclosed in Bradley J. Dworak et al. (2009): "Novel MEA platform with PDMS microtunnels enables the detection of action potential propagation from isolated axons in culture", LAB ON A CHIP 9(3):404-410.

So far the known devices and methods did not prove particular successful in supporting development of new therapeutic intervention for neuronal disorders in human patients. In particular, they often fail to mimic the physiological complexity that characterizes neural networks. As yet, there is no integrated, human relevant, *in vitro* 3D system for assessing efficacy, neurotoxicity or seizure liability based on the inherent physiological characteristics of the neurological system, i.e. electrical activity, tissue architecture, synaptic structures, that is fit for purpose for use in the commercial sector.

Against this background it is an object underlying the invention to provide a new device which allows the interrogation and/or stimulation of neurons in 3D non-adherent networks *in vitro,* thereby creating a platform for studying neural functions, neurodegenerative disorders and testing potentially neuro-active substances. In particular, a device is to be provided which allows both the measuring of action potential propagation and synaptic transmission between connected neurons and the direct electrical stimulation of the neurites, both in the presence and absence of test substances, without interfering with the architecture and integrity of the neurons.

This object is met by providing a device for recording electrical activity of and/or stimulating neural cells in a three-dimensional neural network, comprising:
- a first compartment comprising a three-dimensional hydrogel or fibrous matrix configured to contain neurons and maintain said neurons in said three-dimensional matrix, and
- at least one microchannel extending from said first compartment and having dimensions allowing the extension of neurites from said first compartment into said microchannel and preventing the entry of the soma of neurons into said at least one microchannel,
wherein said at least one microchannel comprises at least one microelectrode embedded therein and arranged to record electrical signals from and/or administer electrical pulses to neurites extending along said at least one microchannel, and wherein said three-dimensional matrix is configured to allow the cultivation of the cells without adhering or contacting the walls or boundary surfaces of the compartment.

The inventors have surprisingly realized that with a device designed in the prescribed manner the measuring of neuronal excitability as well as the electrical stimulation along neurites arising from 3D neuronal architectures will become possible without interfering with the architecture and integrity of the neurons. The device according to the invention will therefore allow the testing of compounds for their neuro-active potential in the context of complex neuronal networks, such as potentially neuropharmacologically active drugs, environmental pollutants and pesticides etc.

According to the invention, the at least one microchannel comprise dimensions allowing the extension of neurites from said first compartment into said at least one microchannel and prevent the entry of the soma of neurons. The skilled person is perfectly aware of the dimensions of the microchannels to fulfill such preconditions as the dimensions of neurons or their neurites and soma are well described in literature. An exemplary however non-binding example of an appropriate microchannel of an embodiment of the invention comprises the following dimensions: approx. 3 µm high, approx. 3 - 4 µm wide, and approx. 100 - 500 µm and preferably approx. 300 µm long.

The at least one microelectrode integrated into the microchannel will allow the measuring or initiation of action potential propagation and synaptic transmission between neurons connected in 3D. Importantly, according to the invention the microelectrode is embedded into the microchannel in such a way that it comes in close vicinity with the neurites present in the microchannel without penetrating or injuring the neurite. The microelectrode can partially extend and/or protrude into the microchannel in such a way that it does not penetrate or injure the neurite. Preferably, however the microelectrode does not extend and/or protrude into the microchannel. Thereby, the microelectrode will not penetrate or injure the neurite. This offers the advantage over other electrophysiological techniques, such as patch-clamp used to record from 3D neuronal structures, of being non-invasive. Therefore, measurements and/or stimulations can be taken or effected repeatedly at any time-point during cultivation.

The "at least one compartment" may be completely enclosed, or may be open on one or more sides.

"At least one microchannel" means that there may be more than one microchannel or even a plurality of microchannels.

According to the invention, a "microelectrode" refers to any device capable of being integrated into a microchannel and recording electrical activity of and/or stimulating, preferably electrically, neural cells. A microelectrode has suitable properties to its function, including low impedance and low noise. The microelectrode may be made of a suitable material for performing its function, including but not limited to gold, platinum, iridium, iridium oxide, titanium nitride, carbon nanotubes, graphene, diamond, a conducting polymer such as poly(3,4-ethylenedioxythiophene) or combinations of these materials. The microelectrode may include a coating which enables or improves its function. The microelectrode may be positioned at different positions inside the microchannel. Further, there may be more than one microelectrode inside a channel. The microelectrode may be positioned on one side of the channel, may enclose the perimeter of the channel, may be smaller than the length of the channel, or it may cover the entire length of a channel.

The at least one microelectrode which is arranged to record electrical signals from neurites is referred to as "recording electrode". The at least one microelectrode which is arranged to administer electrical signals to neurites is referred to as "stimulation electrode". It is to be understood that the same microelectrode can have both functions, i.e. one microelectrode fulfills the activity of both of the recording electrode and the stimulation electrode. However, the recording electrode and the stimulation electrode can be provided as two distinct microelectrodes.

In an embodiment of the invention the device or parts thereof can be made from polymers, glass or ceramic, or a combination thereof. Such glasses include, by way of example, borosilicate glass or fused silica. Such ceramics include, by way of example, quartz, silicon oxide, or silicon nitride. Glasses and ceramics can be patterned by plasma etching, wet etching, and laser etching. Glasses and ceramics have advantages of thermal and chemical resistance. On the other hand, glasses and ceramics suffer from challenging fabrication. Suitable polymers include, by way of example, polydimethylsiloxane (PDMS). PDMS offers several advantages, such as easy fabrication, optical clarity and gas permeability. On the other hand PDMS can variably absorb small hydrophobic compounds, which may lead to changes in bioavailability for some compounds. For this reason alternative polymers such as SU-8 or other epoxy-based negative photoresists may be used to fabricate the device or parts thereof. SU-8 offers advantages such as the ability to fabricate high resolution multilayer structures not possible with other methods. However, SU-8 suffers from challenging fabrication, limited optical transparency and autofluorescence. For this reason alternative polymers such as cyclic olefin copolymers (COC) or cyclic-olefin polymers (COP) may be used to fabricate the device or parts thereof, preferably by injection molding.

According to the invention "three-dimensional network" refers to the spatial connection of neurons in a biocompatible matrix allowing the cultivation of the cells without adhering or contacting the walls or boundary surfaces of the compartment.

The object underlying the invention is herewith completely achieved.

In an embodiment of the invention said device is further comprising a second compartment configured for the cultivation of neurons, wherein a first connecting region comprises the at least one microchannel leading to said second compartment thereby connecting said first compartment with said second compartment.

In this embodiment the neurons were put and cultured in the first compartment, and neurites reach the second compartment via the at least one microchannel. Said first and second compartments may have the same features and characteristics. In particular, the may comprise spatial dimensions allowing the three-dimensional (3D) cultivation of the neurons, i.e. the formation of neuronal networks or circuits, respectively. In a non-binding exemplary embodiment of the invention, for this reason, the first and second compartments have the following dimensions: approx. > 100 µm high, approx. > 300 µm wide, and approx. > 2 mm long.

In a further development of the invention the device is further comprising:
- a third compartment configured for the cultivation of neurons,
- a second connecting region connecting said second and third compartments, said second connecting region comprising the at least one microchannel having dimensions allowing the extension of neurites from said second to said third compartment and preventing the entry of the soma of neurons,
wherein said at least one further microchannel comprises at least one further microelectrode embedded therein and arranged to record electrical signals from and/or administer electrical pulses to neurites extending along said at least one further microchannel.

The features, characteristics and advantages mentioned further above for the first and second compartments, the first connecting region, the at least one microchannel and the microelectrode *mutatis mutandis* apply to the third compartment and the second connecting region, at least one the further microelectrode.

This embodiment of the invention results in the advantage that the measuring of neuronal excitability as well as the electrical stimulation will become possible along more than one but two or more neurons or neurites connected in series along different compartments. Furthermore, in an embodiment of the invention this configuration will allow the provision of a stimulation microelectrode embedded into the microchannel(s) of the first connecting region and of a recording microelectrode embedded into the second connecting region or *vice versa.* Such configuration may help to establish a complex experimental set-up where the influence of test compound on the progression of induced electrical impulses could be examined.

It goes without saying that the device according to the invention may comprise more than three compartments, but multiple compartments, i.e. four, three, five, six, ..., ten, twenty, thirty, ... etc., connected by connecting regions each comprising at least one microchannel.

In another embodiment of the invention the microelectrode is realized and/or comprises a transducer.

This measure has the advantage that a particularly well suited kind of microelectrode is used. A transducer includes the recording and stimulation sites of Complementary Metal-Oxide-Semiconductor (CMOS) devices such as CMOS-MEAs.

In another embodiment of the invention said at least one microchannel and/or said at least one further microchannel comprise multiple microelectrodes.

This measure has the advantage that electrical signals can be recorded and/or electrical pulses can be administered at different sections of the neurites. The multiple microelectrodes may be of the same type, thereby allowing a more precise recording and/or more effective stimulation. The multiple electrodes may also be of different types allowing various signal recordings and/or stimulations.

In another preferred embodiment of the invention said first connecting region comprises a plurality of said microchannel and/or said second connecting region comprises a plurality of said further microchannel.

This embodiment allows the recording and stimulation at numerous neurites in parallel, thereby gaining data which reflect the physiological conditions in the brain in an improved manner.

A "plurality" refers to up to about 50 to about 500 or more microchannels per connecting region.

In another embodiment said dimensions of said microchannel and/or said further microchannel comprise at its/their smallest dimension(s) a diameter of < 5 µm, preferably of about ≤ 4 µm, further preferably of about ≤ 3 µm, further preferably of about ≤ 2 µm, further preferably of about ≤ 1 µm, further preferably of about ≤ 0.5 µm, further preferably of about ≤ 0.4 µm, further preferably of about 0.3 µm, further preferably of about 0.2 µm, and further preferably of about 0.1 µm.

By this measure the constructive preconditions are provided ensuring dimensions allowing the extension of neurites from the compartments into the microchannels but preventing the entry of the soma of neurons.

In an embodiment of the invention multiple devices, e.g. such as about 2 to 12 devices, as characterized herein are assembled, thereby forming a device assembly comprising multiple functional units. This will allow the running of multiple separate assays in parallel, thereby qualifying the invention as a valuable tool for high throughput applications.

In another further development of the invention at least one of said first and second and third compartments comprises an opening on its top, preferably at least one of said first and second and third compartments is open on top.

"Opening or open on top" means that the compartments are accessible from outside. This measure has the advantage that e.g. cell culture media or test substances etc. can be easily added to the cells.

In a preferred further development of the device according to the invention said first and second and said third compartments comprise reservoirs terminal to said channels, further preferably said reservoirs are configured for the seeding of neurons and/or delivery of additives, such as culture media and/or test compounds.

The "reservoir" comprises a volume that is larger than the volume of the channel part of the compartments. It also may comprise an opening on top which is larger than the opening of the compartments. The reservoir therefore facilitates the plating of the neurons into the device and the addition of media, test compounds etc.

In another further development of the device according to the invention said reservoirs comprise a cell seeding area.

The cell seeding area may comprise a physical barrier allowing the defined seeding of the neurons separate from the remaining area of the reservoir. As an example, the cell seeding area may be realized by a subsidence or depression in the base of the reservoirs, e.g. in a central position thereof. Due to its physical separation the cell seeding area also allows the deposition of a scaffold for the growing of the neurons in 3D, such as a hydrogel, which may fill the cell seeding area and may be connected with at least parts of the channels.

In another further development of the invention at least one of said first and second and third compartments comprises a lower sub-compartment and an overlying top sub-compartment adjacent thereto, preferably said microchannels lead to said lower sub-compartment.

The lower and overlying top sub-compartments are physically distinct from each other, while, at the same time, connected with each other. The measure allows, e.g., the filling of the lower compartment with a scaffold for the growing of the neurons in 3D such as a hydrogel, and of the overlying top-compartment with cell culture medium or buffer etc. where test substances may be added to. The test substances may then and diffuse through the medium or buffer and the scaffold to the neurons.

In a preferred configuration the microchannel(s) lead or open out into the lower sub-compartment. This has the advantage that neurites growing from the non-adherent neurons find and extend into the microchannels near to the bottom of the microfluidic part of the device which, in turn, creates the condition for a simple fitting of the microelectrodes from the bottom or the underside, respectively.

In another further development of the invention said lower sub-compartment comprises a width or diameter which is wider than the width or diameter of said top sub-compartment.

By this measure the constructive conditions for a physical delimitation of the lower and overlying sub-compartments are realized. The broadening of the lower sub-compartment over the top compartment facilitates the separation of the scaffold or hydrogel from the overlying medium. A paradigmatic and non-limiting example of the dimensions is as follows. Lower sub-compartment: approx. 400 µm wide; top compartment: approx. 300 µm wide. In an embodiment of the invention the lower sub-compartment may have a height of approx. 150 µm and, independently, the top compartment may have a height of approx. 2 mm.

In a further development of the microfluidic device of the invention at least one of said first and second and, optionally, third compartments comprises at least a transparent material, such as glass.

"Transparent" in this context means optically clear in a way that the cultivated neurons can be visualized with a fit for purpose microscope through the device according to the invention. The optical path runs through the top of the devices, the neurons and the bottom. This measure has the advantage that due to the optical clarity of parts of the compartments and/or the device the acquisition of structural data of such 3D multi-cellular architectures may be captured with sub-cellular resolution, e.g. by confocal microscopy. The transparency may be realized by a glass plate, e.g. a glass MEA configured in a way as to provide the microelectrodes below the microchannel(s). Other transparent materials may be used such as optically clear polymers. It goes without saying that the entire microfluidic device may be made of optically clear materials.

Another subject-matter of the invention relates to a method for examining neurons, comprising the following steps:
- cultivating of neurons in a first compartment in a three-dimensional hydrogel or fibrous matrix such that the neurons are not adhering or contacting the walls or boundary surfaces of said first compartment, wherein at least one microchannel is extending from said first compartment, said at least one microchannel has dimensions allowing the extension of neurites from said first compartment into said at least one microchannel and preventing the entry of the soma of neurons into said at least one microchannel;
- letting the neurites of said neurons growing along said at least one microchannel,
- recording electrical signals from and/or administering electrical pulses to said neurites growing along said at least one microchannel by at least one recording microelectrode and/or at least one stimulation microelectrode embedded in said at least one microchannel.

The features, characteristics, further developments and advantages mentioned for the device according to the invention apply likewise to the method according to the invention.

In a further development of the method according to the invention said first compartment is connected via a first connecting region with a second compartment, said first connecting region comprising said at least one microchannel leading to said second compartment thereby connecting said first compartment with said second compartment, and the neurites of said neurons are allowed to grow through said at least one microchannel towards said second compartment.

In an alternative embodiment of the method according to the invention the neurons can be cultivated in the second and/or third compartment. The neurons will then pass through the first connecting region or, optionally, the second connecting region to said first and/or second and/or third compartment and/or *vice versa.* It is to be understood that, in another embodiment of the invention, the neurons can be cultivated in all of the compartments in parallel.

In the method according to the invention said cultivating is carried out in a scaffold allowing a three-dimensional cultivation of said neurons, namely in a hydrogel or a fibrous matrix.

This measure has the advantage that preferred structures are provided which favor the three-dimensional cultivation of the neurons.

In another embodiment of the method according to the invention in addition to or instead of said neurons non-neuronal cells are cultivated.

Such measure has the advantage that a situation is established resembling even more the physiological situation where non-neuronal cells take over other functions such as support functions.

In another embodiment the three-dimensional matrix includes neurospheres and/or minibrains and/or brain organoids.

This measure takes advantage of using more heterogeneous structures comprised by the 3D neuronal architectures.

In another development of the method according to the invention additives, preferably test compounds, are added to said first and/or second or, optionally third compartment.

It is to be understood that the before-mentioned features and those to be mentioned in the following cannot only be used in the combination indicated in the respective case, but also in other combinations or in an isolated manner without departing from the scope of the invention.

The features mentioned in the specific embodiments are also features of the invention in general, which are not only applicable in the respective embodiment but also in an isolated manner in the context of any embodiment of the invention.

The invention is also described and explained in further detail by referring to the following drawings:
- Fig. 1: shows a plan view on top of an embodiment of a microfluidic device assembly according to the invention;
- Fig. 2: shows a cross section along the line II-II of the embodiment depicted in Fig. 1;
- Fig. 3: shows a cross section along the line III-III of the embodiment depicted in Fig. 1;
- Fig. 4: shows a magnification of the microchannels area of the embodiment depicted in Fig. 3;
- Fig. 5: shows a plan view on the bottom of one of the functional units or microfluidic device of the embodiment shown in Fig. 3;
- Fig. 6: shows a detail view on the bottom side of connecting region and the microchannels with integrated microelectrodes of the embodiment shown in Fig. 5;
- Fig. 7: shows a bird's eye view onto an embodiment of a microfluidic device assembly;
- Fig. 8: illustrates a prototype of the device according to the invention, in which neurons are cultivated within a three-dimensional matrix in a bottom sub-compartment with a separate top sub-compartment for liquid perfusion. Microchannels and microelectrodes (not shown here) according to the invention allow recording and/or stimulation of the neurons in a three-dimensional matrix.
- Fig. 9: shows a scanning electron microscope image of the prototype depicted in Fig. 8.
- Fig. 10: illustrates an embodiment of the device according to the invention from a bird's-eye-view.
- Fig. 11: shows a side sectional view of the embodiment of Fig. 10.
- Fig. 12: shows another embodiment of the device according to the invention.
- Fig. 13: illustrates a further embodiment of the device according to the invention.
- Fig. 14: illustrates an embodiment of the connecting region.
- Fig. 15: illustrates various embodiments of microchannels.
- Fig. 16: shows a confocal 3D image of GFP-labelled primary neurons cultivated in an embodiment of the device assembly according to the invention;
- Fig. 17: shows a confocal 3D image of a single GFP-labelled human iPSC-derived neuron growing in the second compartment of an embodiment of the device assembly according to the invention;
- Fig. 18: shows a high resolution detail of a single neurite of the neuron depicted in Fig. 17;
- Fig. 19: shows (left and middle) a differential interference contrast (DIC) microscopic image of human iPSC-derived neurons grown in 3D on an embodiment of a microfluidic device according to the invention. Right panel shows action potentials recorded by microelectrodes integrated into microchannels.

### Embodiments

### 1. The microfluidic device

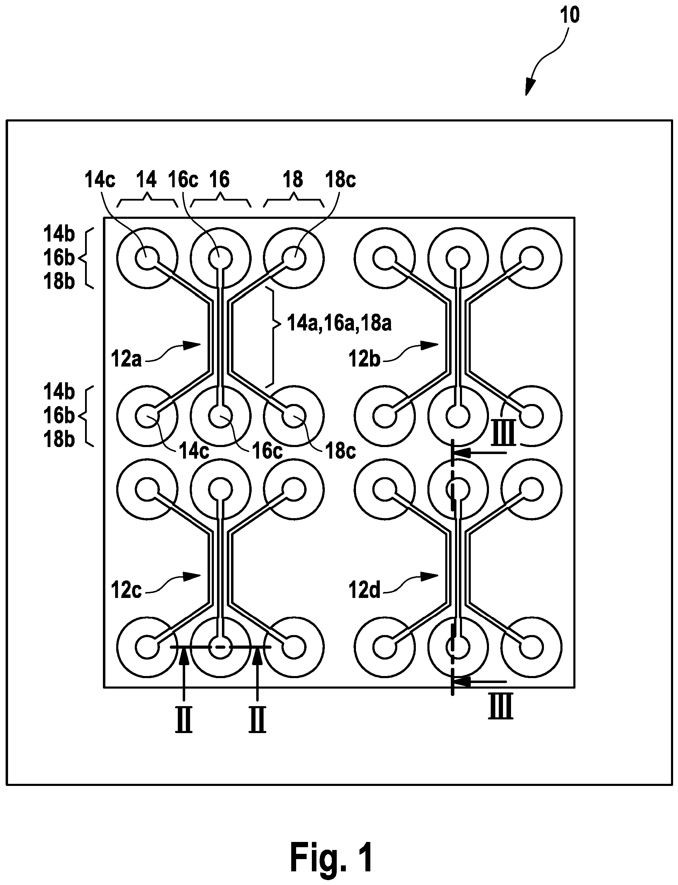

In Fig. 1 a plan view on top of an embodiment of a device assembly is shown under the reference sign 10. The device assembly 10 may be manufactured by means of photolithography using a polymer such as SU-8 or by means of injection moulding using polymers such as cyclic olefin copolymer (COC) or cyclin olefin polymer (COP) or by means of selective laser etching using glass. In the shown embodiment the device assembly 10 has the dimensions of 32 mm (width) x 32 mm (length) x 3 mm (height). The device assembly 10 comprises four corresponding devices or functional units or 12a, 12b, 12c, 12c, separated from each other, which allow the cultivation of four independent neuron cell cultures. Each of the units 12a, 12b, 12c, 12d comprises a first 14, a second 16, and a third compartment 18 each configured for the cultivation of neurons. The first, second, and third compartments 14, 16, 18 each comprise a channel section 14a, 16a, 18a and a terminal reservoir 14b, 16b, 18b which are connected with each other. Each of said terminal reservoirs 14b, 16b, 18b comprises a cell seeding area 14c, 16c, 18c.

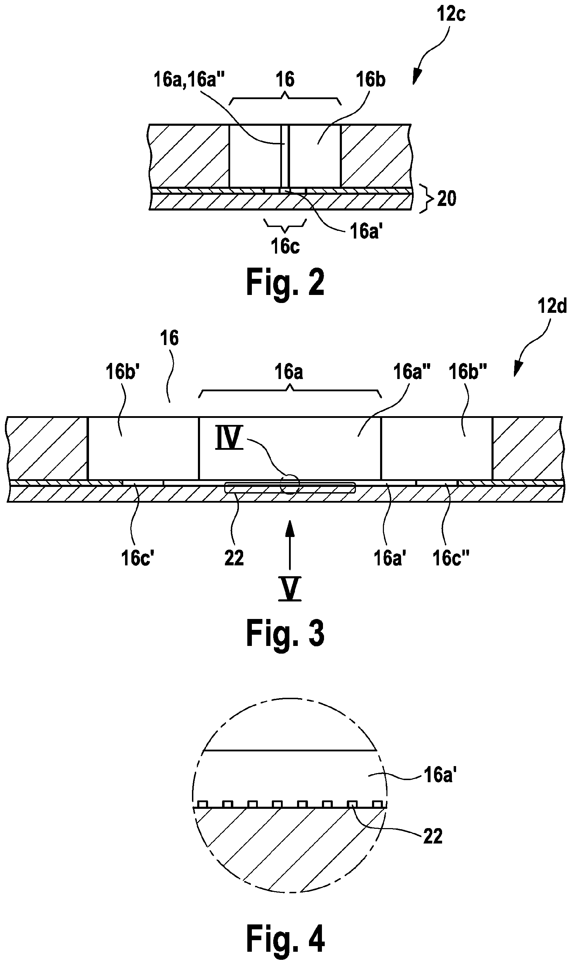

In Fig. 2 a cross section along the line II-II of the second compartment 16 of the functional unit 12c is depicted. It illustrates that the terminal reservoir 16b is positioned onto a base plate 20 which may form an integrated part of the device assembly 10 but may also be a glass MEA affixed to the upper unit forming a microfluidic part of the device in a liquid-tight manner. The cell seeding area 16c is embedded into the base plate 20. The terminal reservoir 16b and the cell seeding area 16c lead to the channel section 16a.

As it can be inferred from Fig. 2 the channel section 16a and the terminal reservoir 16b are both open on top which allows the addition of culture media and test compounds into the device assembly 10. It is also shown that the channel section 16a comprises a lower sub-compartment 16a' and an overlying top sub-compartment 16a" adjacent thereto. The lower sub-compartment 16a' comprises a diameter or a channel width, respectively, which is wider than the diameter or the channel width, respectively, of said top sub-compartment 16a". Both the overlying top sub-compartment 16a" and the lower sub-compartment 16a' open into the terminal reservoir 16b. The lower sub-compartment 16a' extends to the cell seeding area 16c as a result of a subsidence in the base plate 20.

In Fig. 3 a cross section along the line III-III of the functional unit 12d as shown in Fig. 1 is depicted. It illustrates the arrangement of the channel section 16a, the lower sub-compartment 16a' and the overlying top sub-compartment 16a", the terminal reservoirs 16b and the cell seeding areas 16c which - on that representation - are located at the left 16b', 16c' and the right termini 16b", 16c" of the channel section 16a. It is also again shown that both the overlying top sub-compartment 16a" and the lower sub-compartment 16a' open into the terminal reservoirs 16b' and 16b". The lower sub-compartment 16a' extends to the cell seeding areas 16c', 16c" as a result of a recess in the base plate 20. Microchannels 22 open into the lower sub-compartment 16a', in particular in that part which is outside of the terminal reservoirs 16b' and 16b". The microchannels 22 are provided in a connecting region 24 (not shown here) which connect the second 16 and the neighboring first compartment 14 and extend into the lower sub-compartment 14a' of the channel section 14a of the first compartment 14 (not shown here). Fig. 4 shows a magnification of the microchannels 22 which are provided in the connecting region 24 which connects lower sub-compartment 16a' with lower sub-compartment 14a'.

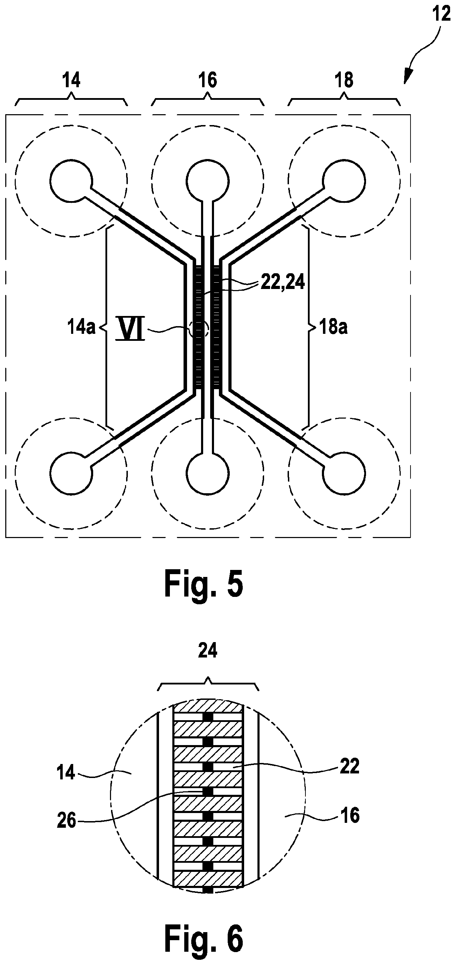

Fig. 5 shows a plan view on the bottom of the embodiment of a functional unit 12 of the device assembly 10. Shown in black are the connecting regions 24 which comprise the microchannels 22 which connect the second 16 with the neighboring first 14 and with the neighboring third compartment 18, and extend into the respective lower sub-compartments 14a', 18a' (not shown here) of the channel sections 14a, 18a of the first and third compartments 14, 18.

Fig. 6 shows a detail view on the bottom side of connecting region 24 which connects the second 16 with the neighboring first 14 compartment. In the depicted embodiment each of the microchannels 22 comprises a microelectrode 26 embedded therein. The microelectrodes 26 may be integrated into a glass MEA substrate below each of the microchannels 22 and arranged to record electrical signals from or administer electrical pulses to neurites extending along said microchannels 22. Therefore, the microelectrodes 26 include recording and stimulation microelectrodes.

In a preferred but non-limiting embodiment of the device assembly 10 each connecting region 24 comprises 32 microelectrodes 26, each functional unit 12, therefore, comprises 64 microelectrodes 26 and the entire device assembly 10 comprises 256 microelectrodes 26.

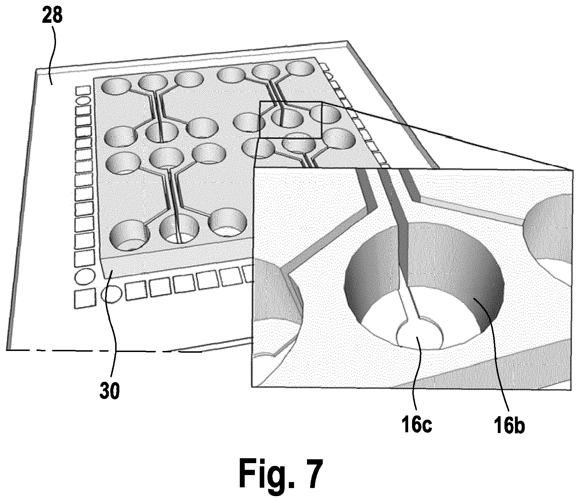

Fig. 7 shows a bird's eye view onto an embodiment of a device assembly 10 and a magnification of the terminal reservoir 16b and the cell seeding area 16c of the second compartment 16. In this embodiment the bottom of the microfluidic device assembly 10 is consisting of a glass MEA 28 comprising the microelectrodes 26 (not shown here) affixed to the upper microfluidic part 30 in a liquid-tight manner.

For cultivation the cells are seeded in the cell seeding areas 16c of the second compartment 16 in a hydrogel scaffold which fills the lower sub-compartments 14a', 16a', 18a' up to the border to the top sub-compartments 14a", 16a", 18a". Neurites can extend into the neighboring first 14 and third compartment 18 by growing through the microchannels 22. The microelectrodes 26 integrated below each of the microchannels 22 record action potentials along single neurites or may apply electrical pulses thereon.

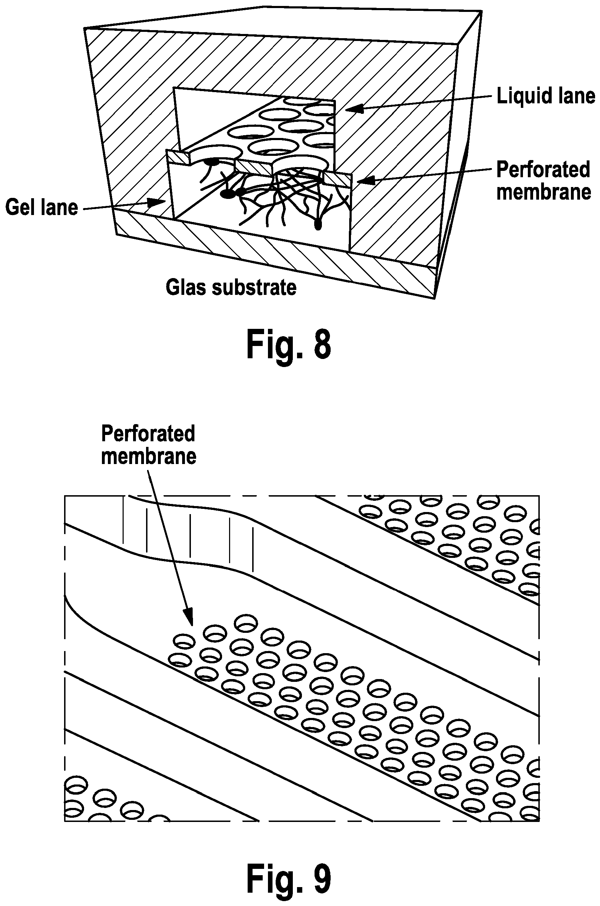

Fig. 8 illustrates a prototype of the device according to the invention where microstructures were fabricated directly on glass substrates by employing epoxy-based negative resists such as SU-8 and a UV lithographic process. Additional thicker layers are then laminated on top by using dry film resists (DFR). It is mainly composed by two microfluidic compartments, one on top of each other, separated by a perforated thin DFR. Fig. 9 shows an SEM image of said perforated membrane fabricated by the inventors using lamination of a dry film resist. Membrane thickness is 20 µm. Perforations are 30 µm in diameter. The bottom compartment was used for seeding neurons dispersed in a 3D matrix such as hydrogel, while the top compartment was later filled with liquid media. Diffusion through the perforated membrane assure that nutrients, gases and catabolites are continuously exchanged between the liquid and the gel compartments, providing the required conditions for long term cultivation of neurons in 3D and application of test compounds. Microchannels 22 (not shown here) are provided in a connecting region 24 (not shown here) and comprise microelectrodes 26 (not shown here). The microchannels allow extension of neurites from neurons dispersed in 3D and thereby allow recording and/or stimulation of their electrical activity by the microelectrodes.

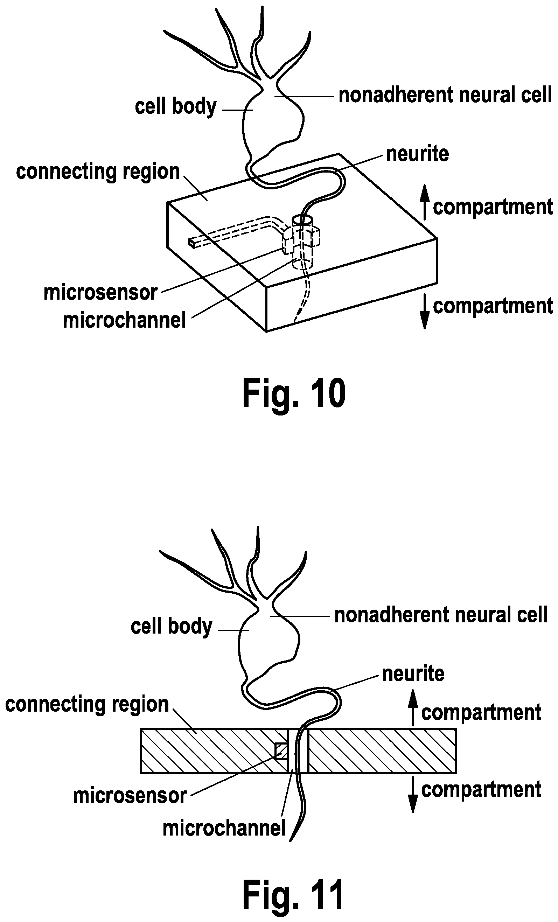

In Fig. 10 an embodiment of the device according to the invention is depicted from a bird's-eye-view. In Fig. 11 a side view of this embodiment of the device according to the invention is depicted.

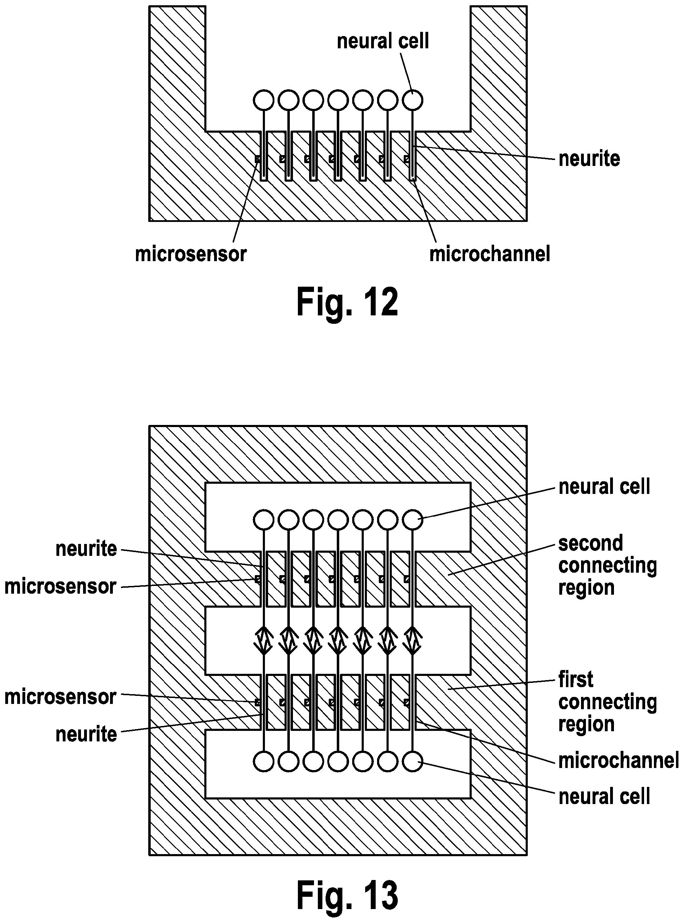

In Fig. 12 another embodiment of the device according to the invention is depicted. In this embodiment an open compartment is provided with its lower connecting region containing seven closed microchannels with single microelectrodes. Neurons extend neurites into the microchannels.

In Fig. 13 a further embodiment of the device according to the invention is illustrated. In this embodiment of three compartments separated by two connecting regions, each containing seven microchannels with single microelectrodes. Different neural cell types are cultured in the top and bottom compartments. Their cell bodies are constrained to their respective compartments. The cells communicate by synaptic connections after extending neurites through the channels into the central compartment.

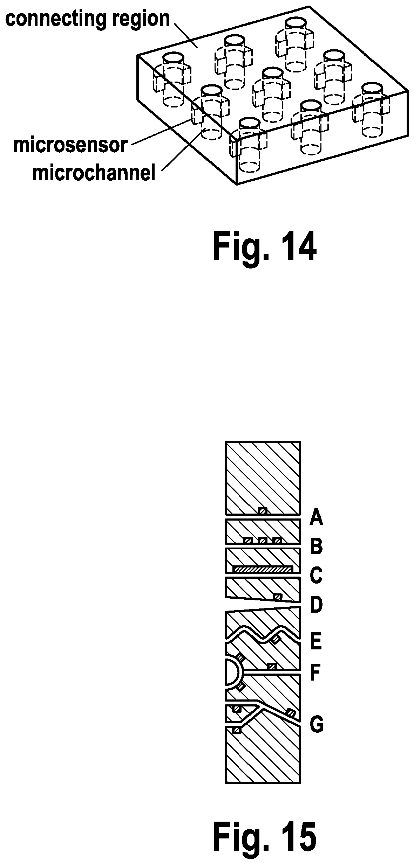

Fig. 14 illustrates the design of the connecting region. In a bird's-eye-view multiple microchannels in a connecting region are depicted, each microchannel having a single microelectrode.

In Fig. 15 various embodiments of microchannels are illustrated such as a straight channel with single microelectrode (A), a straight channel with multiple microelectrodes (B), a channel with a long microelectrode (C), a channel with varying width (D), a curved channel (E), split channels with multiple microelectrodes (F, G).

### 2. Fabrication and evaluation

*General:* An MEA having a footprint of 49 mm x 49 mm and containing 256 integrated recording microelectrodes, arranged in a 4 x 64 matrix, is designed and fabricated. Patterning of microelectrodes-aligned microchannels (3 µm high, 7.5 µm wide, 300 µm long) on the upper surface of MEA is achieved by photolithography of SU-8.

At the same time, a range of different designs, materials and fabrication processes is evaluated for their capacity to consistently produce the microfluidic part of the device assembly (32mm wide, 32 mm long, 3 mm high) compatible with the goal of growing cells in 3D. In particular, materials like COC and COP, considered industry standards for biopharmaceutical applications, are tested for injection moulding. Materials like glass are tested for selective laser etching. The material is tested for the successive MEA bonding process (by solvent bonding or thermal bonding or chemical bonding) as well as for the purpose of culturing viable 3D neuronal networks.

After bonding, the new hybrid MEA/microfluidic device is subjected to biological testing and functional characterization.

*Design and Mastering:* The design of the microfluidic device assembly can be transferred to a design for mass manufacturing. After taking care of typical design rules for injection moulding and adapting the design accordingly, metal inserts can be manufactured by micro-milling. Depending on the complexity a new base mould might be required during the development.

*Injection moulding:* After realising the insert for the mould, injection tests can be performed with different materials e.g. different grades of COC or COP. This will enable to test and to compare different properties of the moulded material for the succeeding bonding process as well as in the final application.

*Selective laser etching:* The design of the microfluidic device assembly can be transferred to a design for mass manufacturing. After taking care of typical design rules for selective laser etching and adapting the design accordingly, glass microfluidic parts can be produced by selective laser etching.

*Bonding:* The MEA can be manufactured such that the upper surface will be micro-patterned by a photolithographic process based on SU8. During the bonding task the moulded part may be permanently attached to this SU8 layer. Bonding of injection moulded parts may be done by solvent bonding or thermal bonding or chemical bonding. A bonding process of COC/COP or glass to SU8 is used which will not destroy the micro-features on SU8 and has a sufficient bonding strength for the application.

*Characterisation and Application tests:* Characterisation and application tests are performed in parallel. Characterisation includes: geometrical measurements by scanning electron microscopy (SEM), profilometry and transparency checks. Application tests can start in parallel to the bonding tests. This will give first hints about the bonding strength and how this is affected by the different strategies used for bonding.

*Combining structural and functional readouts:* Due to the optical clarity of the device, capturing structural data by live microscopy will allow reconstructing 3D neuronal morphologies in detail. A recent study performed by the inventors has shown how by using high resolution confocal microscopy it was possible to identify fine neuronal ultra-structures, such as dendritic spines, in live 3D neurons cultured on matrigel scaffolds. Recording neuronal activity by microelectrodes offers the advantage, over other electrophysiological techniques, of being non-invasive. Therefore, measurements can be taken repeatedly at any time-point during cultivation. In combination to imaging experiments this offers the unique possibility to combine continuous acquisition of structural and functional parameters from 3D cultured neurons into a single live assay.

*Platform Validation:* This will be carried out in order to verify the capacity of the device according to the invention to identify and predict a range of interactions between specific classes of compounds and neuronal pathways. Electrophysiological and structural read-outs can be both employed to monitor the responses to a small ad selected group of reference compounds. This will ensure that the most relevant molecules will be selected, according to a list of criteria such as: i) chemical structure, ii) applications routes (nervous system drugs, other-organ drugs, pesticides) and iii) range of engaged cellular pathways. For testing purposes, the compounds can be divided into 3 calibration sets, containing 5 compounds each (3 positive and 2 negative controls), for the purpose of identifying subtypes of response patterns:
1. Efficacy Set: neuropharmacologically active drugs.
2. Safety Set: molecules with demonstrated seizurogenic activity.
3. Toxicity Set: environmental pollutants and pesticides with a defined mechanism of action.

*Throughput:* Each microfluidic device assembly, with a footprint of only 49 mm x 49 mm, may contain a total of 256 microelectrodes, arranged in a matrix of up to 12 x 21 microelectrodes. This will ensure multiple independent experiments on each functional unit, each having enough microelectrodes to sample a large number of neurons. A range of compound concentrations plus control solutions could be run on each chip, providing enough through-put to support rapid screening campaigns.

*Cost-efficiency:* Due to the compact size of the microfluidic device assembly, running each single assay will only require a few thousand cells and less than 100 µl of test compounds. This means, for example, that one vial of human iPS cells, containing 1-2 million cells on average, will be sufficient for up to 60 independent measurements. This, considering the very high prices of iPS cells, will contribute to keep costs at an acceptable level for this type of assay.

*Bio-compatibility:* Most microfluidic devices currently used to create organs-on-chip are made in PDMS. Although this material offers several advantages, such as easy fabrication, optical clarity and gas permeability, on the other hand PDMS can variably absorb small hydrophobic compounds, which may lead to changes in bioavailability for some compounds. For this reason alternative materials such as COC or COP or glass, gold standards in biopharmaceutical testing, can be used to fabricate the microfluidic chip. Although this will require a more complex process for production of the device and thermal or solvent or adhesive bonding to attach it to the MEA substrate, the use of such materials is considered advantageous considering its intended screening purposes.

*Long-term 3D cultivation:* Achieving long-term cell survival in hydrogel scaffolds requires a controlled flow of culture media through the microfluidic chip. The media is in fact used to exchange gases between air and the gel, to provide nutrients and remove metabolites, to apply compounds during drug testing. These features will be implemented in the microfluidic device assembly design, based on a channel containing a hydrogel lane (400 µm wide, 150 µm high) at the bottom of the device (lower sub-compartment) and a liquid lane (300 µm wide, up to 2 mm high) on top of the gel lane (top sub-compartment). In this way media/solutions/drugs can be easily applied/replaced at the top liquid lane via reservoirs and from here they can rapidly diffuse to the cells contained in the bottom gel lane.

*Handling:* Liquid handling and drug applications will be done as described above, using an open system that does not require any specific equipment, as all liquids in the top lane will move between the reservoirs passing through open channels having low resistance. This provides the option to add robotic control of all liquid handling steps, as eventually required for screening purposes.

*Cross-platform functionalities:* With a footprint of only 49 mm x 49 mm and an optically-clear glass bottom, the microfluidic device according to the invention will be available for capturing simultaneously electrophysiological and imaging data using standard MEA recording apparatus and confocal/scanning disk microscopy. Microelectrode arrays can be designed compatible to the format of recording hardware/software produced by commercial providers.

### 3. Cultivation and examination of nerve cells

The inventors have successfully tested the microfluidic device assembly for its capability to cultivate and examine neurons in the context of 3D neuronal networks.

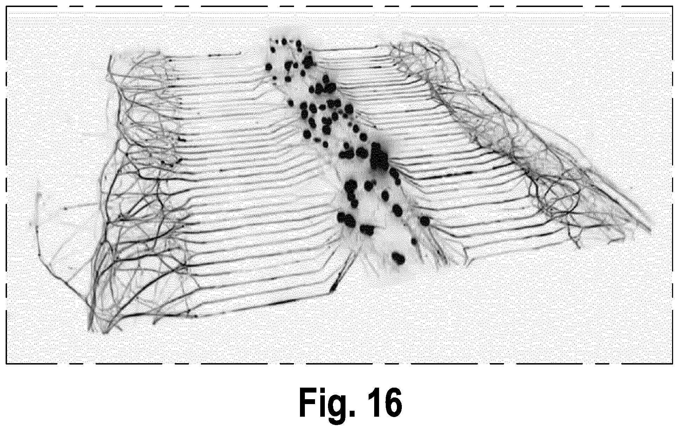

Fig. 16 shows a live confocal 3D imaging of GFP-labelled primary neurons in a microfluidic device according to the invention. From a central compartment containing the soma, several neurites extend through the microfluidic channels at the bottom of the device, until they reach the lateral compartments and continue growing in 3D.

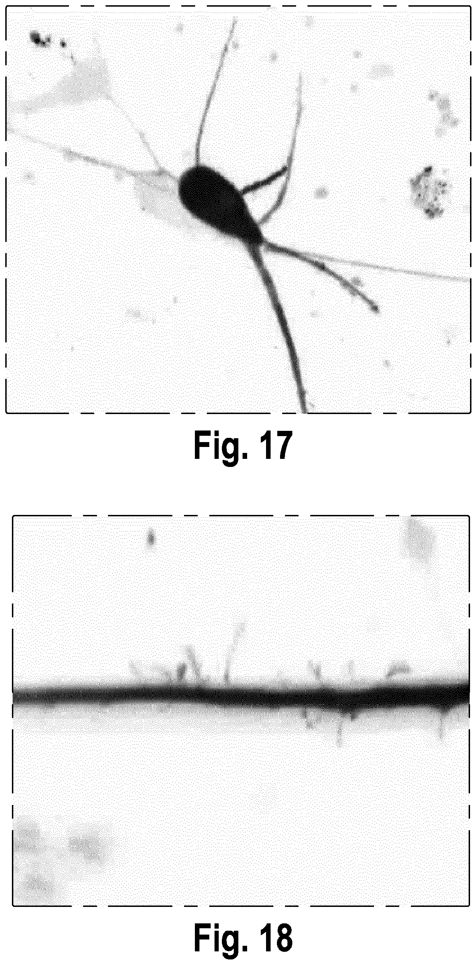

*Emulating 3D brain networks:* To partially reconstruct the complex multi-cellular structure of the human brain, commercially available neuronal (excitatory and inhibitory) and glial (astrocytes) cells derived from human iPSCs are grown in 3D inside the microfluidic device assembly, using a range of biomimetic hydrogel scaffolds (bio-derived or synthetic). In order to evaluate cell viability, neuronal outgrowth and 3D architectures obtained using different microfluidic designs, materials and cell types, live imaging experiments are carried out by labelling cells with a range of genetically-encoded fluorescent proteins. Using this approach the inventors obtained data with various prototype devices showing GFP-labelled neurons growing extensively in 3D within few days from seeding. Fig. 17 shows a live confocal 3D imaging of GFP-labelled human iPSC-derived neurons growing in the central compartment of a microfluidic device assembly according to the invention. Neurites can be seen growing extensively in all directions.

Fig. 18 shows a higher resolution digital reconstruction of previous image shown in Fig. 17. A single neurite (less than 1 µm thick) is observed in 3D from different angles, with several dendritic spines being clearly visible.

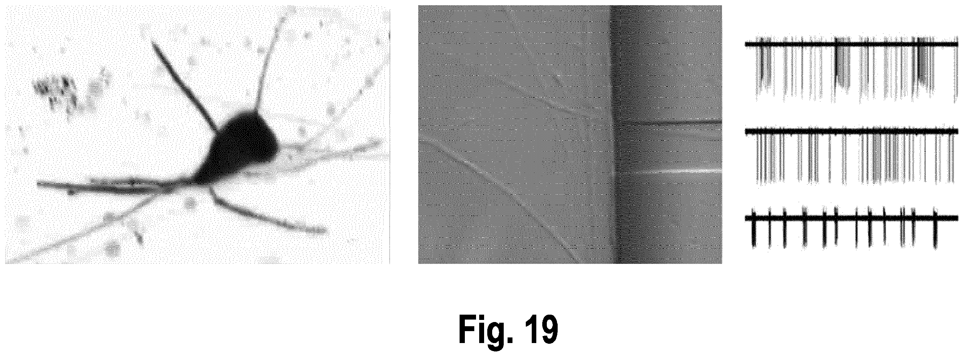

*Recording neuronal activity from 3D networks:* As neurites elongate through the dedicated microchannels at the bottom of the microfluidic device assembly according to the invention, microelectrodes integrated below each microchannel will allow measuring action potential propagation and synaptic transmission between neurons connected in 3D. Different microelectrodes sizes and positions within the microchannels are evaluated to identify the ideal dimensions to obtain the best signal-to-noise ratio. The inventors obtained proof-of-concept results showing that iPSC-derived neurons cultured in 3D (Fig. 19, left) are capable to grow neurites through the microchannels (Fig. 19, middle), where integrated microelectrodes could clearly measure from single neurites individual action potentials originating from spontaneously active neurons (Fig. 19, right).

As can be seen from Fig. 19 which shows differential interference contrast (DIC) live imaging of human iPSC-derived neurons grown in 3D on a microfluidic device according to the invention, cells can be observed at different positions along the z axis. In left panel cell bodies are mainly visible, while in *middle* panel the neurites can be observed entering the microchannels at the bottom of the device. *Right* trace shows action potentials recorded from microelectrodes (not visible here) integrated at the bottom of the microchannels.

## Claims

1. A device (10) for recording electrical activity of and/or stimulating neural cells in a three-dimensional neural network, comprising:
- a first compartment (14) comprising a three-dimensional hydrogel or fibrous matrix configured to contain neurons and to maintain said neurons in said three-dimensional matrix, and
- at least one microchannel (22) extending from said first compartment (14) and having dimensions allowing the extension of neurites from said first compartment (14) into said microchannel (22) and preventing the entry of the soma of neurons into said at least one microchannel (22),
**characterized in that** said at least one microchannel (22) comprises at least one microelectrode (26) embedded therein and arranged to record electrical signals from and/or administer electrical pulses to neurites extending along said at least one microchannel (22), and
that said three-dimensional matrix is configured to allow the cultivation of the cells without adhering or contacting the walls or boundary surfaces of the compartment (14).

2. The device (10) of claim 1, further comprising a second compartment (16), wherein a first connecting region (24) comprises the at least one microchannel (22) leading to said second compartment thereby connecting said first compartment (14) with said second compartment (16).

3. The device (10) of claim 1 or 2 further comprising:
- a third compartment (18),
- a second connecting region (24) connecting said second (16) and third compartments (18), said second connecting region (24) comprising at least one further microchannel (22) having dimensions allowing the passing-through of neurites from said second (16) to said third compartment (18) and preventing the entrance of the soma of neurons into said microchannel(s) (22),
wherein said at least one further microchannel (22) comprises at least one further microelectrode (26) embedded therein and arranged to record electrical signals from and/or administer electrical pulses to neurites extending along said at least one further microchannel (22),
preferably wherein said second connecting region (24) comprises a plurality of said further microchannel (22).

4. The device (10) of any of the preceding claims, **characterized in that** the microelectrode(s) (26) comprise(s) a transducer.

5. The device (10) of any of the preceding claims, **characterized in that** said at least one microchannel (22) and/or said at least one further microchannel (22) comprise multiple microelectrodes (26).

6. The device of any of the preceding claims, **characterized in that** said first connecting region (24) comprises a plurality of said microchannel (22).

7. The device (10) of any of the preceding claims, **characterized in that** said dimensions of said microchannel (22) and/or said further microchannel (22) at its/their smallest dimension(s) are < 5 µm, preferably of about ≤ 4 µm, further preferably of about ≤ 3 µm, further preferably of about ≤ 2 µm, further preferably of about ≤ 1 µm, further preferably of about ≤ 0.5 µm, further preferably of about ≤ 0.4 µm, further preferably of about 0.3 µm, further preferably of about 0.2 µm, and further preferably of about 0.1 µm.

8. The device (10) of any of the preceding claims, **characterized in that** at least one of said first (14) and, if applicable, second (16) and third compartments (18) comprises transparent material, preferably glass.

9. A method for examining neurons, comprising the following steps:
- cultivating of neurons in a first compartment (14) in a three-dimensional hydrogel or fibrous matrix such that the neurons are not adhering or contacting the walls or boundary surfaces of said first compartment (14), wherein at least one microchannel (22) is extending from said first compartment (14), said at least one microchannel (22) has dimensions allowing the extension of neurites from said first compartment (14) into said at least one microchannel (22) and preventing the entry of the soma of neurons into said at least one microchannel (22);
- letting the neurites of said neurons grow along said at least one microchannel (22),
- recording electrical signals from and/or administering electrical pulses to said neurites growing along said at least one microchannel by at least one recording microelectrode (26) and/or at least one stimulation microelectrode (26) embedded in said at least one microchannel (22).

10. The method of claim 9, **characterized in that** said first compartment (14) is connected via a first connecting region (24) with a second compartment (16), said first connecting region (24) comprising said at least one microchannel (22) leading to said second compartment (16) thereby connecting said first compartment (14) with said second compartment (16), and the neurites of said neurons are allowed to grow through said at least one microchannel (22) towards said second compartment (16).

11. The method of any of claims 9 and 10, **characterized in that** in addition to said neurons non-neuronal cells are cultivated.

12. The method of any of claims 9 to 11, **characterized in that** the three-dimensional matrix include neurospheres and/or brain organoids.

13. The method of any of claims 9 to 12, **characterized in that** additives, preferably test compounds, are added to said first and/or second compartment.

## Patentansprüche

1. Vorrichtung (10) zum Aufzeichnen von elektrischer Aktivität und/oder zum Stimulieren von neuronalen Zellen in einem dreidimensionalen neuronalen Netzwerk, die Folgendes aufweist:
- ein erstes Kompartiment (14), das ein(e) dreidimensionale(s) Hydrogel oder Fasermatrix aufweist, das/die ausgebildet ist, um Neuronen zu enthalten und die Neuronen in der dreidimensionalen Matrix aufrecht zu halten, und
- zumindest einen Mikrokanal (22), der sich von dem ersten Kompartiment (14) aus erstreckt und Abmessungen aufweist, die die Erstreckung von Neuriten aus dem ersten Kompartiment (14) in den Mikrokanal (22) ermöglichen, und die den Eintritt des Somas von Neuronen in den zumindest einen Mikrokanal (22) verhindern,
**dadurch gekennzeichnet, dass** der zumindest eine Mikrokanal (22) zumindest eine darin eingebettete Mikroelektrode (26) aufweist, die so angeordnet ist, dass sie elektrische Signale von Neuriten, die sich entlang des zumindest einen Mikrokanals (22) erstrecken, aufzeichnet und/oder ihnen elektrische Impulse zuführt, und
dass die dreidimensionale Matrix ausgebildet ist, um die Kultivierung der Zellen zu ermöglichen, ohne an den Wänden oder Grenzflächen des Kompartiments (14) zu haften oder diese zu kontaktieren.

2. Vorrichtung (10) nach Anspruch 1, die ferner ein zweites Kompartiment (16) aufweist, wobei ein erster Verbindungsbereich (24) den zumindest einen Mikrokanal (22) aufweist, der zu dem zweiten Kompartiment führt, wodurch das erste Kompartiment (14) mit dem zweiten Kompartiment (16) verbunden wird.

3. Vorrichtung (10) nach Anspruch 1 oder 2, die ferner Folgendes aufweist, nämlich
- ein drittes Kompartiment (18),
- einen zweiten Verbindungsbereich (24), der das zweite (16) und das dritte Kompartiment (18) verbindet, wobei der zweite Verbindungsbereich (24) zumindest einen weiteren Mikrokanal (22) aufweist, der Abmessungen aufweist, die den Durchtritt von Neuriten vom zweiten (16) zum dritten Kompartiment (18) ermöglichen, und die den Eintritt des Somas von Neuronen in den/die Mikrokanal/Mikrokanäle (22) verhindern,
wobei der zumindest eine weitere Mikrokanal (22) zumindest eine weitere Mikroelektrode (26) aufweist, die darin eingebettet und so angeordnet ist, dass sie elektrische Signale von Neuriten, die sich entlang des zumindest einen weiteren Mikrokanals (22) erstrecken, aufzeichnet und/oder ihnen elektrische Impulse zuführt,
wobei der zweite Verbindungsbereich (24) vorzugsweise eine Vielzahl der weiteren Mikrokanäle (22) aufweist.

4. Vorrichtung (10) nach einem der vorhergehenden Ansprüche, **dadurch gekennzeichnet, dass** die Mikroelektrode(n) (26) einen Transducer aufweist (aufweisen).

5. Vorrichtung (10) nach einem der vorhergehenden Ansprüche, **dadurch gekennzeichnet, dass** der zumindest eine Mikrokanal (22) und/oder der zumindest eine weitere Mikrokanal (22) multiple Mikroelektroden (26) aufweist.

6. Vorrichtung nach einem der vorhergehenden Ansprüche, **dadurch gekennzeichnet, dass** der erste Verbindungsbereich (24) eine Vielzahl der Mikrokanäle (22) aufweist.

7. Vorrichtung (10) nach einem der vorhergehenden Ansprüche, **dadurch gekennzeichnet, dass** die Abmessungen des Mikrokanals (22) und/oder des weiteren Mikrokanals (22) bei ihrer/ihren kleinsten Abmessung(en) < 5 µm, vorzugsweise etwa ≤ 4 µm, weiter vorzugsweise etwa ≤ 3 µm, weiter vorzugsweise etwa ≤ 2 µm, weiter vorzugsweise etwa ≤ 1 µm, weiter vorzugsweise etwa ≤ 0,5 µm, weiter vorzugsweise etwa ≤ 0,4 µm, weiter vorzugsweise etwa 0,3 µm, weiter vorzugsweise etwa 0,2 µm und weiter vorzugsweise etwa 0,1 µm betragen.

8. Vorrichtung (10) nach einem der vorhergehenden Ansprüche, **dadurch gekennzeichnet, dass** zumindest eine der ersten (14) und gegebenenfalls zweiten (16) und dritten Kompartimente (18) ein transparentes Material, vorzugsweise Glas, aufweist.

9. Verfahren zur Untersuchung von Neuronen, das die folgenden Schritte aufweist:
- Kultivieren von Neuronen in einem ersten Kompartiment (14) in einem/einer dreidimensionalen Hydrogel oder Fasermatrix, so dass die Neuronen nicht an den Wänden oder Grenzflächen des ersten Kompartiments (14) haften oder damit in Kontakt kommen, wobei sich zumindest ein Mikrokanal (22) von dem ersten Kompartiment (14) aus erstreckt, der mindestens eine Mikrokanal (22) Abmessungen aufweist, die die Erstreckung von Neuriten aus dem ersten Kompartiment (14) in den zumindest einen Mikrokanal (22) erlauben, und die den Eintritt des Somas von Neuronen in den zumindest einen Mikrokanal (22) verhindern;
- Wachsenlassen der Neuriten der Neuronen entlang des zumindest einen Mikrokanals (22),
- Aufzeichnen elektrischer Signale von den und/oder Verabreichen elektrischer Impulse an die Neuriten, die entlang des mindestens einen Mikrokanals wachsen, durch zumindest eine Aufzeichnungsmikroelektrode (26) und/oder zumindest eine Stimulationsmikroelektrode (26), die in dem zumindest einen Mikrokanal (22) eingebettet sind.

10. Verfahren nach Anspruch 9, **dadurch gekennzeichnet, dass** das erste Kompartiment (14) über einen ersten Verbindungsbereich (24) mit einem zweiten Kompartiment (16) verbunden ist, wobei der erste Verbindungsbereich (24) den zumindest einen Mikrokanal (22) aufweist, der zu dem zweiten Kompartiment (16) führt und dadurch das erste Kompartiment (14) mit dem zweiten Kompartiment (16) verbindet, und die Neuriten der Neuronen durch den zumindest einen Mikrokanal (22) in Richtung des zweiten Kompartiments (16) wachsen können.

11. Verfahren nach einem der Ansprüche 9 und 10, **dadurch gekennzeichnet, dass** zusätzlich zu den Neuronen nicht-neuronale Zellen kultiviert werden.

12. Verfahren nach einem der Ansprüche 9 bis 11, **dadurch gekennzeichnet, dass** die dreidimensionale Matrix Neurosphären und/oder Hirnorganoide aufweist.

13. Verfahren nach einem der Ansprüche 9 bis 12, **dadurch gekennzeichnet, dass** dem ersten und/oder zweiten Kompartiment Zusatzstoffe, vorzugsweise Testverbindungen, zugesetzt werden.

## Revendications

1. Dispositif (10) d'enregistrement d'activité électrique et/ou de stimulation des cellules neurales dans un réseau neuronal tridimensionnel, comprenant :
- un premier compartiment (14) comprenant un hydrogel tridimensionnel ou une matrice fibreuse configurée pour contenir des neurones et pour maintenir lesdits neurones dans ladite matrice tridimensionnelle, et
- au moins un microcanal (22) s'étendant à partir dudit premier compartiment (14) et présentant des dimensions permettant l'extension de neurites à partir dudit premier compartiment (14) dans ledit microcanal (22) et empêchant l'entrée du soma de neurones dans ledit au moins un microcanal (22),
**caractérisé en ce que** ledit au moins un microcanal (22) comprend au moins une microélectrode (26) qui y est intégrée et agencée pour enregistrer des signaux à électriques provenant de et/ou pour administrer des impulsions électriques aux neurites s'étendant le long dudit au moins un microcanal (22), et
que ladite matrice tridimensionnelle est configurée pour permettre la culture des cellules sans adhérer ni entrer en contact avec les parois ou les surfaces limites du compartiment (14).

2. Dispositif (10) de la revendication 1, comprenant en outre un deuxième compartiment (16), dans lequel une première région de liaison (24) comprend au moins un microcanal (22) conduisant audit deuxième compartiment, reliant ainsi ledit premier compartiment (14) audit deuxième compartiment (16).

3. Dispositif (10) de la revendication 1 ou 2, comprenant en outre :
- un troisième compartiment (18),
- une deuxième région de liaison (24) reliant lesdits deuxième (16) et troisième compartiments (18), ladite deuxième région de liaison (24) comprenant au moins un microcanal supplémentaire (22) présentant des dimensions permettant le passage de neurites dudit deuxième compartiment (16) audit troisième compartiment (18) et empêchant l'entrée du soma de neurones dans ledit/lesdits microcanal/microcanaux (22),
dans lequel ledit au moins un microcanal supplémentaire (22) comprend au moins une microélectrode supplémentaire (26) qui y est intégrée et agencée pour enregistrer des signaux électriques provenant de et/ou pour administrer des impulsions électriques aux neurites s'étendant le long dudit au moins un microcanal supplémentaire (22),
de préférence dans lequel ladite deuxième région de liaison (24) comprend une pluralité desdits microcanaux supplémentaires (22).

4. Dispositif (10) de l'une des revendications précédentes, **caractérisé en ce que** la/les microélectrode(s) (26) comprend/comprennent un transducteur.

5. Dispositif (10) de l'une des revendications précédentes, **caractérisé en ce que** ledit au moins un microcanal (22) et/ou ledit au moins un microcanal supplémentaire (22) comprennent de multiples microélectrodes (26).

6. Dispositif de l'une des revendications précédentes, **caractérisé en ce que** ladite première région de liaison (24) comprend une pluralité desdits microcanaux (22).

7. Dispositif (10) de l'une des revendications précédentes, **caractérisé en ce que** lesdites dimensions dudit microcanal (22) et/ou dudit microcanal supplémentaire (22) dans sa/leurs plus petite(s) dimension(s) sont < 5 µm, de préférence environ ≤ 4 µm, de préférence encore environ ≤ 3 µm, de préférence encore environ ≤ 2 µm, de préférence encore environ ≤ 1 µm, de préférence encore environ ≤ 0,5 µm, de préférence encore environ ≤ 0,4 µm, de préférence encore environ de 0,3 µm, de préférence encore environ de 0,2 µm et de préférence encore environ de 0,1 µm.

8. Dispositif (10) de l'une des revendications précédentes, **caractérisé en ce qu'**au moins l'un parmi lesdits premier (14) et, le cas échéant, deuxième (16) et troisième (18) compartiments est constitué d'un matériau transparent, de préférence du verre.

9. Procédé d'examen de neurones, comprenant les étapes suivantes :
- la culture de neurones dans un premier compartiment (14) dans un hydrogel tridimensionnel ou une matrice fibreuse de sorte que les neurones n'adhèrent pas ou n'entrent pas en contact avec les parois ou les surfaces limites dudit premier compartiment (14), dans lequel au moins un microcanal (22) s'étend dudit premier compartiment (14), ledit au moins un microcanal (22) présente des dimensions permettant l'extension de neurites à partir dudit premier compartiment (14) dans ledit au moins un microcanal (22) et empêchant l'entrée du soma de neurones dans ledit au moins un microcanal (22) ;
- la croissance des neurites desdits neurones le long dudit au moins un microcanal (22),
- l'enregistrement de signaux électriques provenant de et/ou l'administration d'impulsions électriques auxdits neurites qui croissent le long dudit moins un microcanal par au moins une microélectrode d'enregistrement (26) et/ou au moins une microélectrode de stimulation (26) intégrée dans ledit au moins un microcanal (22).

10. Procédé de la revendication 9, **caractérisé en ce que** ledit premier compartiment (14) est relié par l'intermédiaire d'une première région de liaison (24) à un deuxième compartiment (16), ladite première région de liaison (24) comprenant au moins un microcanal (22) conduisant audit deuxième compartiment (16) reliant ainsi ledit premier compartiment (14) audit deuxième compartiment (16), et les neurites desdits neurones sont autorisés à croître à travers ledit au moins un microcanal (22) vers ledit deuxième compartiment (16).

11. Procédé de l'une des revendications 9 et 10, **caractérisé en ce qu'**en plus desdits neurones, des cellules non neuronales sont cultivées.

12. Procédé de l'une des revendications 9 à 11, **caractérisé en ce que** la matrice tridimensionnelle comprend des neurosphères et/ou des organoïdes cérébraux.

13. Procédé de l'une des revendications 9 à 12, **caractérisé en ce que** des additifs, de préférence des composés d'essai, sont ajoutés audit premier et/ou deuxième compartiment.
